# EUROPEAN PATENT APPLICATION

(11) **EP 1 878 460 A1**
(43) Date of publication of application: **16.01.2008**
(21) Application number: 06746322.4
(22) Date of filing: 02.05.2006
(51) Int. Cl.: A61M 16/10

(54) **SILENCER AND OXYGEN CONCENTRATION DEVICE USING THE SAME**

(30) Priority: 06.05.2005 JP 2005134842
(71) Applicant: Teijin Pharma Limited, Tokyo 100-0011 (JP)
(72) Inventor: KUWABARA, Katsunori c/o Teijin Pharma Limited,, Hinodecho,Iwakuni-shi,Yamaguchi;740001 (JP)
(74) Representative: Bachelin, Karl Martin Raimund
(86) International application number: PCT/JP2006/309525
(87) International publication number: WO 2006/121139

(57) **Abstract**

Provided are an expansion-type silencer (10) including a substantially rectangular parallelepiped-shaped expansion chamber (16) surrounded by six partition walls and having a suction port (20) and an exhaust port (22) which are formed in the partition walls, and an oxygen concentrator using the expansion-type silencer (10). The expansion-type silencer (10) has a rib (24) extending from one of the partition walls of the expansion chamber (16) formed with the exhaust port (22) toward the interior of the expansion chamber (16) while being in contact with both of a pair of parallel partition walls adjacent to the one of partition walls. The rib (24) is formed with an air passage (26) for providing communication between the exhaust port (22) and the expansion chamber (16).

## Description

### TECHNICAL FIELD

The present invention relates to a silencer, and in particular to a medical oxygen concentrator for supplying oxygen-concentrated air to a user such as a patient with a respiratory disease and a silencer for reducing noise during operation of the concentrator.

### BACKGROUND ART

In Oxygen inhalation therapy, high-concentration oxygen is inhaled by patients with chronic respiratory disease, such as pulmonary emphysema, pulmonary tuberculosis sequela or chronic bronchitis. The oxygen inhalation therapy is a treatment in which a patient with chronic respiratory disease is made to inhale a high concentration oxygen gas or an oxygen-concentrated gas. Means used for supplying a high concentration oxygen gas or oxygen concentrated gas to the patients may include a high-pressure oxygen cylinder, a liquid oxygen cylinder and an oxygen concentrator. However, an oxygen concentrator has been increasingly used since it can be used continuously for long period of time and is easy to use.

The oxygen concentrator is a device which can separate and concentrate oxygen in the air. A separation film type of oxygen concentrator, which uses an oxygen permeable film to separate oxygen, and an adsorption type of oxygen concentrator, which has one or more adsorption beds filled with an adsorbent capable of selectively adsorbing nitrogen are widely known as oxygen concentrators. However, an adsorption-type oxygen concentrator is used in wider applications because it can provide oxygen at a higher concentration. In particular, a variable pressure adsorption-type oxygen concentrator using a compressor as a pressure changing device is common.

In the oxygen concentrator of the variable pressure adsorption-type, high concentration oxygen is generally obtained by repeating, in a predetermined cycle, a adsorption process for supplying compressed air from a compressor to one or more adsorption beds filled with an adsorbent for selectively adsorbing nitrogen and a desorption process for desorbing nitrogen from the adsorption bed by decompressing the interior of the adsorption bed.

This oxygen concentrator is placed relatively near the patient, and is basically used continuously over twenty-four hours, regardless of when a patient eats and drinks or sleeps. Therefore, noise generated from the oxygen concentrator directly may be heard by the patient or his/her family, thereby making them feel uncomfortable. Especially during sleep, noise has a greater affect on the patient or his/her family, and may disturb the sleep of the patient or his/her family and adversely affect their mental health. Factors of noise generation from the variable pressure adsorption-type of oxygen concentrator include solid-borne sound generated from the compressor for changing pressure, sounds produced by the suction and exhaust of the compressor, operational sound produced by a motor for driving the compressor, purge gas flow sound produced by the nitrogen adsorption bed, and operational sound produced by a fan for cooling the interior of the device. Especially, sounds attributable to the compressor include the solid-borne sound generated by the compressor itself and sound produced by the suction and exhaust of the compressor account for a major percentage of the total amount of noise.

In the conventional oxygen concentrator, a silencer called a hollow-type or expansion-type is used to reduce sound of flowing gas, such as suction sound, exhaust sound or various purge sounds from the compressor. For example, Japanese Unexamined Patent Publication No. 2001-120662 discloses an example of an oxygen concentrator using an expansion-type silencer to reduce a purge sound. This expansion-type silencer includes an expansion chamber, an inflow pipe for introducing purge gas into the expansion chamber, and an outflow pipe for discharging the purge gas from the expansion chamber, wherein an effect of reducing the sound level is achieved by a change in the acoustic impedance at the abruptly enlarged portion at the entrance of the expansion chamber from the inflow pipe and the abruptly reduced portion at the entrance of the outflow pipe from the expansion chamber. This expansion-type silencer generally has a cylindrical shape, and inflow and outflow pipes constituted by round pipes are connected to the upper and lower surfaces of the silencer, so that the noises are reduced by taking advantage of the ratio of the sectional area between the inflow and outflow pipes and the cylindrical expansion chamber. In order to improve the silencing effect, a silencer of an insertion pipe-type also has been developed in which parts of the inflow and outflow pipes are inserted and projected into the expansion chamber.

Also, as described in Japanese Unexamined Patent Publication No. 10-245203, a silencer having an expansion chamber formed into a rectangular parallelepiped shape has been developed.

As described above, it is important to quiet noise from the compressor and any other noise sources in the oxygen concentrator. In addition, a reduction of the size and weight of the silencer is required to reduce the size and weight of the oxygen concentrator. However, in the silencer of the insertion pipe-type described above, the larger the ratio between the sectional areas of the inflow and outflow pipes (the diameters of the inflow and outflow pipes) and the sectional area of the expansion chamber, the higher the rate at which generated sound is attenuated. Thus, the length of the expansion chamber is related to the frequency of the sound which is desired to be reduced. Therefore, the physical size of the expansion chamber is determined based on the frequency band of the sound to be reduced and the attenuation rate, which is one disincentive in reducing the size and weight of the body of the low-noise oxygen concentrator. For example, a conventional expansion-type silencer is generally cylindrical. Therefore, once the sectional area of the expansion chamber is determined, the thickness thereof is determined. In addition, dead space is created around the silencer, and as a result, large installation space is required.

Also, the expansion chamber portion and the insertion pipe portion have a circular pipe shape. Therefore, a draft of the pipe portion required to produce the silencer by resin molding is difficult to secure and the processing accuracy is low, thereby making it difficult to produce the silencer by resin molding. For this reason, the insertion pipe portion and the expansion chamber portion are required to be molded separately from each other and assembled together. Thus, three or more parts are required, resulting in a low mass, productivity. Further, the machining of the inside of the insertion pipe is difficult. In addition, in order to reduce noises, the pressure loss, i.e. the power consumption of the power source, and the silencing effect are required to be adjusted by changing the length and the inner diameter of the insertion pipe, thereby posing a problem that design work is difficult.

Thus, in order to enhance the silencing effect of the silencer while at the same time reducing the size, weight and cost, various measures such as changing the material of the silencer from metal to resin and reducing the thickness of the partition walls of the silencer are required. However, in the current expansion-type silencer having the inflow and outflow pipes combined with the cylindrical expansion chamber, it is impossible to achieve a sufficient size reduction and silencing effect without increasing pressure loss.

### DISCLOSURE OF THE INVENTION

Accordingly, it is an object of the present invention to solve the aforementioned problems of the conventional silencer and oxygen concentrator and to reduce the size of the silencer and thus realize a small, light, low-noise, low-power-consumption oxygen concentrator.

According to a first aspect of the present invention, there is provided a silencer which includes a substantially rectangular parallelepiped-shaped expansion chamber surrounded by six partition walls and having a suction port and an exhaust port which are formed in the partition walls, wherein the silencer further includes a rib extending from one of partition walls of the expansion chamber formed with the exhaust toward the interior of the expansion chamber while being in contact with both of a pair of parallel partition walls adjacent to the one of partition walls, the rib formed with an air passage for providing communication between the exhaust port and the expansion chamber.

In the above silencer, the expansion chamber is preferably formed by a box portion having four side walls, a bottom wall and the rib integrated with each other, and a lid portion closely contacting the four side walls and the rib, and the air passage is preferably formed by a groove formed on the rib and the lid portion closely contacting the rib.

One or both of the suction port and the exhaust port are preferably formed by an orifice having a sectional area progressively decreasing along a direction of flow.

A front chamber having a communication port communicating with the outside is preferably provided outside the exhaust port of the expansion chamber. In this case, the silencer may further include a filter in the front chamber or in the expansion chamber.

Further, according to a second aspect of the present invention, there is provided an expansion-type silencer which includes a substantially rectangular parallelepiped-shaped internal chamber surrounded by six partition walls, the internal chamber divided into a first chamber and a second chamber by a separation wall, the first chamber formed at an outer wall thereof with a suction port, the separation wall formed with an exhaust port, wherein the silencer further includes a rib extending from the separation wall formed with the exhaust port toward the interior of the first chamber while being in contact with both of a pair of parallel partition walls adjacent to the separation wall, the rib formed with an air passage connecting to the exhaust port for providing communication between the first and second chambers.

Further, according to a third aspect of the present invention, there is provided an oxygen concentrator which includes a supply pipe, an exhaust pipe and an expansion-type silencer connected to one or both of the supply pipe and the exhaust pipe, the oxygen concentrator separating oxygen from raw air supplied from the supply pipe thereby to generate an oxygen concentrated gas and discharging the exhaust gas after separation of oxygen from the exhaust pipe, wherein the expansion-type silencer includes a substantially rectangular parallelepiped-shaped expansion chamber surrounded by six partition walls and having a suction port and an exhaust port which are formed in the partition walls, and a rib extending from one of the partition walls of the expansion chamber formed with the exhaust port toward the interior of the expansion chamber while being in contact with both of a pair of parallel partition walls adjacent to the one of the partition walls, the rib formed with an air passage for providing communication between the exhaust port and the expansion chamber.

In the above oxygen concentrator, the expansion chamber is preferably formed by a box portion having four side walls, a bottom wall and the rib integrated with each other, and a lid portion closely contacting the four side walls and the rib, and the air passage is preferably formed by a groove formed on the rib and the lid portion closely contacting the rib.

One or both of the suction port and the exhaust port are preferably formed by an orifice having a sectional area progressively decreasing along a direction of flow.

A front chamber having a communication port communicating with the outside is preferably provided outside the exhaust port of the expansion chamber. In this case, the silencer may further include a filter in the front chamber or in the expansion chamber.

The silencer according to the present invention is substantially rectangular parallelepiped-shaped and, as compared with the cylindrical silencer, can minimize a dead space when installed in the oxygen concentrator. Also, the provision of the rib in the expansion chamber of the silencer can reduce the thickness of the outer wall of the expansion chamber, thereby reducing the weight of the silencer. Further, the air pipe formed on the rib can fulfill a function of an insertion pipe, thereby achieving a noise reduction. Therefore, the oxygen concentrator equipped with this silencer and used for home oxygen treatment can reduce the suction and exhaust sounds from the compressor during operation, and at the same time reduce the size, weight and production cost.

### BRIEF DESCRIPTION OF THE DRAWINGS

The above and other objects, features and advantages of the present invention will be described below in more detail based on preferred embodiments of the present invention with reference to the accompanying drawings, in which:
Fig. 1A is an exploded view of a silencer according to the present invention;
Fig. 1B is a sectional view of the silencer taken along line 1B-1B in Fig. 1A;
Figs. 2A to 2C are schematic diagrams showing some examples of a rib of the silencer according to the present invention;
Figs. 3A to 3C are schematic diagrams showing some examples of positions of the suction port and the exhaust port of the silencer according to the present invention;
Fig. 4 is a partly cutaway view of the interior of the silencer with a filter according to the present invention; and
Fig. 5 is a schematic diagram showing a general configuration of an oxygen concentrator of variable pressure type equipped with the silencer according to the present invention.

### BEST MODE FOR CARRYING OUT THE INVENTION

Preferred embodiments of the present invention will be described below with reference to the drawings.

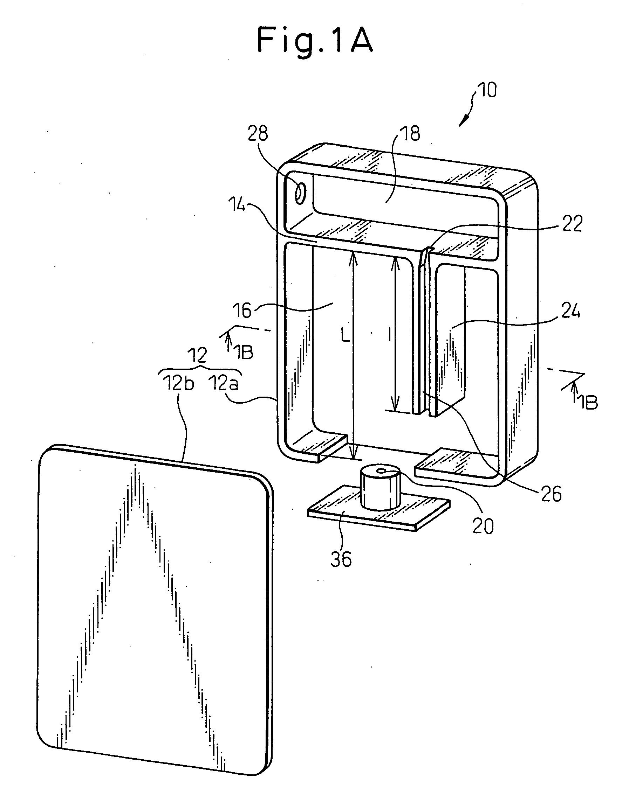

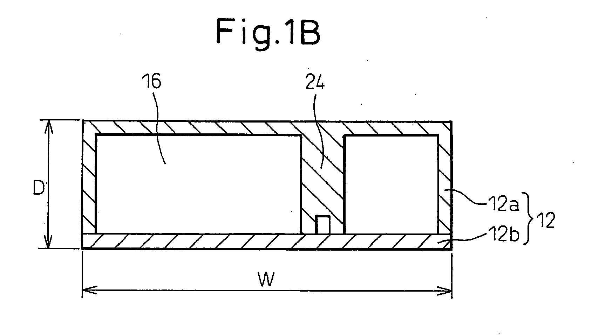

Referring to Figs. 1A and 1B, an expansion-type silencer 10 according to the present invention includes a substantially rectangular parallelepiped-shaped housing 12. The housing 12 includes a parallelepiped-shaped box portion 12a configured of four peripheral walls and a bottom wall with one side surface open, and a rectangular lid portion 12b mounted on the open side surface of the box portion 12a. The box portion 12a and the lid portion 12b are fixed for example by welding to form the housing 12. The internal space of the substantially rectangular parallelepiped-shaped housing 12 is divided into a first chamber and a second chamber by a separation wall 14. The first chamber constitutes an expansion chamber 16, while the second chamber constitutes a front chamber 18. Also, the wall of the expansion chamber 16 is formed with a suction port 20, and the separation wall 14 between the expansion chamber 16 and the front chamber 18 is formed with an exhaust port 22. Further, a rib 24 extending inwardly from the exhaust port 22 of the separation wall 14 while being kept in contact with both the lid portion 12b and the bottom wall of the box portion 12a is provided in the expansion chamber 16. The rib 24 is formed with an air passage for providing communication between the exhaust port 22 and the expansion chamber 16. The air passage 26 of the rib 24 is preferably formed by forming a groove connected to the exhaust port 22 on the side surface of the rib 24 facing the lid portion 12b and welding the lid portion 12b on the side surface of the rib 24.

As described above, the substantially rectangular parallelepiped shape of the housing 12 makes it possible to prevent dead space from being generated around the silencer 10 when the silencer 10 is connected to the oxygen concentrator or the like and minimize the installation space for the silencer 10. Specifically, in the expansion-type silencer 10, the ratio of the sectional area between the expansion chamber 16 and the inflow pipe connected thereto determines the attenuation rate. Therefore, if the same sectional area of the expansion chamber is secured, the installation of the cylindrical silencer, for example, having a diameter of 10 mm would require an installation space of 10 mm x 10 mm, while the substantial rectangular parallelepiped-shaped silencer 10 having the same sectional area can be installed in an installation space of 10 mm x 7.85 mm.

Also, in order to reduce the weight of the silencer 10, the thickness of the housing 12 of the silencer 10 is required to be reduced. However, if the wall of the expansion chamber 16 becomes thin as the result of reducing the thickness of the housing 12, the strength of the wall of the expansion chamber 16 will be reduced and the wall itself will easily vibrate. This can generate a new cause of increased noise. Especially in the case where the expansion chamber 16 of the silencer 10 is in the shape of a flat rectangular parallelepiped, the housing 12 can be easily vibrated by the pulsation of the air flow or the like. In contrast, if the thickness of the wall is thinner, the silencer 10 according to the present invention can suppress the vibration by the rib 24 formed in the expansion chamber 16, thereby preventing the generation of noises due to the vibration. Further, since the presence of the rib 24 increases the strength of the housing 12, the thickness of the housing 12 can be further reduced from the viewpoint of strength and the effect of suppressing the deformation which otherwise would occur due to the draft in the resin molding process can be achieved.

The air passage 26 formed on the rib 24 fulfils a function of an insertion pipe. The insertion pipe is a pipe (a supply pipe and/or the exhaust pipe) leading to the expansion chamber 16 which is extended so as to be projected into the interior of the expansion chamber 16, and generally provides a silencing effect higher than a pipe simply connected to the expansion chamber 16. Therefore, according to the silencer 10 of the present invention, silencing can be enhanced by the air passage 26 formed on the rib 24 in the expansion chamber 16.

As described above, the rib 24 provided in the expansion chamber 16 and having the air passage 26 functions as an insertion pipe and a reinforcing member for preventing the vibration of the expansion chamber 16, thereby achieving two kinds of silencing effects at the same time.

The housing 12 of the silencer 10 can be made using an appropriate material such as metal or resin, and is preferably made from resin such as acrylonitrile butadiene styrene (ABS) or polypropylene (PP) in order to reduce weight and cost.

Also, the housing 12 is configured of two component members, i.e. the box portion 12a and the lid portion 12b. Therefore, the silencer 10 can be easily produced by die molding these two components and assembling them together, thereby contributing to the reduction of the production cost for the silencer 10.

In the case where the air passage 26 is formed by forming a groove on the rib 24, various shapes including circle, rectangle, U- and V- shapes can be employed as the shape of the cross section of the groove of the rib 24. However, since the ratio of the sectional area between the groove and the expansion chamber 16 greatly affects the noise reduction effect, the shape and size of the groove are required to be determined in consideration of the relation with the frequency range of the noise to be silenced. Also, although a longer air passage 26 formed on the groove of the rib 24 provides a higher silencing effect, it poses a problem of increasing a pressure loss at the same time. Therefore, the length of the groove or the length of the rib 24 is preferably at least one half of the length of the expansion chamber 16, and more preferably 3/4 of the length of the expansion chamber 16. In this case, the compressor noise in the frequency range most difficult to reduce, i.e. the frequency range of 800 to 1600 Hz, can be reduced, and at the same time, the noise in the frequency range of 400 to 800 Hz also can be reduced by the increased pressure loss.

The suction port 20 or the exhaust port 22 of the expansion chamber 16 of the silencer 10 according to the present invention is preferably formed as a tapered orifice with the sectional area thereof decreasing along the direction of flow. The tapered orifice is useful especially for reducing the sound of air flow not less than 1000 Hz. Also, by changing the shape and size of the orifice, a great silencing effect can be realized without changing the shape or size of the groove of the rib 24 causing pressure loss.

Further, the expansion chamber 16 functions as a buffer tank. Specifically, the larger volume of the expansion chamber 16 can reduce the effect of the pulsation of the supplied air more largely, thereby providing a greater silencing effect.

The front chamber 18 is formed with an external communication port 28 for providing communication with the outside. The front chamber 18 not only has the effect of silencing the sound of air flow generated in the exhaust port 22 of the expansion chamber 16, but also serves as a connector for connecting to external piping, thereby making it possible to freely design the direction in which the silencer 10 is connected to the piping. Further, in the case where the gas is discharged into the atmosphere through the front chamber 18, the provision of a plurality of the external communication ports 28 can reduce the pressure loss.

In the silencer 10 shown in Figs. 1A and 1B, the internal space of the housing 12 is separated by the separation wall 14 to form the expansion chamber 16 and the front chamber 18. However, only the expansion chamber 16 may be formed in the housing 12 without front chamber 18. Also, the front chamber 18 may be formed by welding a separate housing to the expansion chamber 16. However, in terms of the production cost, it is advantageous to manufacture the box portion 12a, defining the expansion chamber 16 and the front chamber 18, as an integrated molded component, and therefore the structure as shown in Figs. 1A and 1B is preferable.

### [Embodiments]

A silencer according to an embodiment of the present invention will be described below, which is suitable for maintaining a sufficient silencing effect of the compressor while at the same time suppressing the suction gas/exhaust gas pressure loss at not more than 5 kPa when the silencer is attached to the compressor, in the case where the compressor has a suction gas flow rate of 18 L/minute and an exhaust gas flow rate of 16 L/minute.

The box portion 12a and the lid portion 12b of the housing 12 are made from ABS resin or PP resin in order to reduce the weight. Also, a dimension of each part is designed in accordance with a space for installing the silencer such that the volume of the expansion chamber 16 is 165 cm³. The silencer 10 shown in Figs. 1A and 1B, for example, is made to have the thickness D of 35 mm, the width W of 90 mm and the length L of 80 mm of the expansion chamber 16. In this case, the length 1 of the rib 24 in the expansion chamber 16 is preferably 60 mm so as to be 3/4 of the length L of the expansion chamber 16. This rib 24 is formed with an air passage 26 equivalent to a round pipe having an inner diameter of 5 mm in order to allow the air passage 26 to function as an insertion pipe. In this embodiment, the air passage 26 is formed by a groove, formed on an end surface facing the lid portion 12b of the rib 24 and having a quadrangular cross section, and the lid portion 12b closely contacting the groove. The suction port 20 is formed on a connector part 36 mounted on the side wall of the box portion 12a, and the exhaust port 22 is formed on the separation wall 14 for separating the expansion chamber 16 and the front chamber 18 from each other, at the outlet of the groove of the rib 24. Both the suction port 20 and the exhaust port 22 are formed as tapered orifices in which a sectional area of a flow passage equivalent to a round pipe having an inner diameter of 5 mm is reduced along the direction of flow to a sectional area of a flow passage equivalent to a round pipe having an inner diameter of 3 mm. The front chamber 18 is provided at the outlet of the exhaust port 22, and an external communication port 28 is provided on the left side surface of the front chamber 18 in Fig. 1B.

This silencer 10 is generally configured of the two members, i.e. the rectangular lid portion 12b and the box portion 12a having the expansion chamber 16, the rib 24 formed with the groove and the front chamber 18. These two members are finally thermally welded to each other. In this way, a compact, flat silencer 10 having a thickness of 35 mm is manufactured by resin molding. As described above, except for the connector part 36, the silencer 10 can be manufactured of two members, and therefore a silencer which can keep production costs low and can be mass produced can be realized. The connector part 36 may of course be formed to be integrated with the box portion 12a.

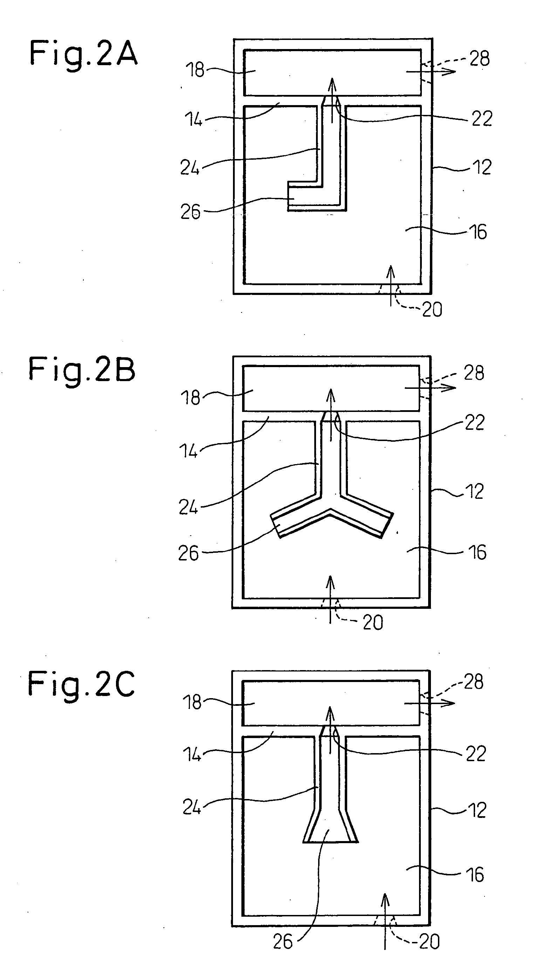

In the embodiment shown in Figs. 1A and 1B, the rib 24 extends in a straight. However, as shown in Figs. 2A to 2C, the rib 24 can have a bent shape (Fig. 2A), a forked shape (Fig. 2B) or a shape having a flared end (Fig. 2C).

The air passage 26 (the portion corresponding to the insertion pipe) is formed by the lid portion 12b and the groove formed on the side surface of the rib 24 facing the lid portion 12b. Therefore, by changing the shape of the rib 24 as described above, the shape of the route of the air passage 26 can also be changed. Further, the flow passage area of the air passage 26 and the shape of the orifice forming the exhaust port 22 can be freely changed, and therefore the silencing effect can be enhanced without a notable change in pressure loss.

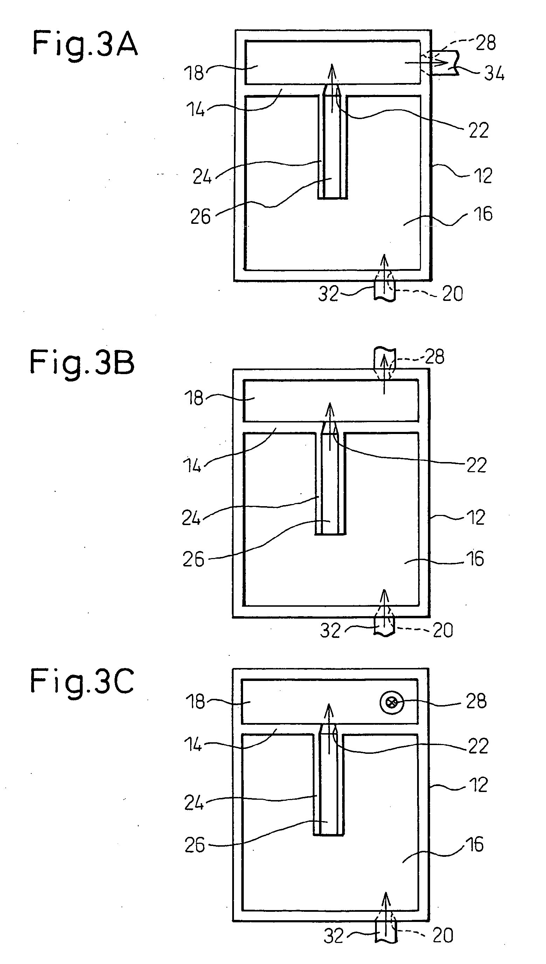

Also, the provision of the front chamber 18 can expand a design freedom of directions in which an inlet-side pipe 32 (a pipe connected to the suction port 20) and an outlet-side pipe 34 (a pipe connected to the external communication port 28 of the front chamber 18) of the silencer 10 are connected to the silencer 10. As shown in Fig. 3A, for example, the external communication port 28 may be formed on the right side surface of the front chamber 18 as viewed in the figure, so that the outlet-side pipe 34 can be connected to the silencer 10 in the direction of 90 degrees with regard to the inlet-side pipe 32 in the plane of the figure. Alternatively, as shown in Fig. 3B, the external communication port 28 may be formed on the upper side surface of the front chamber 18 as viewed in the figure, so that the outlet-side pipe 34 can be connected to the silencer 10 in the direction parallel to the inlet-side pipe 32. Alternatively, as shown in Fig. 3C, the external communication port 28 may be formed on the bottom surface of the front chamber 18, so that the outlet-side pipe 34 can be connected to the silencer 10 in the direction perpendicular to the inlet-side pipe 32 and the plane of the figure.

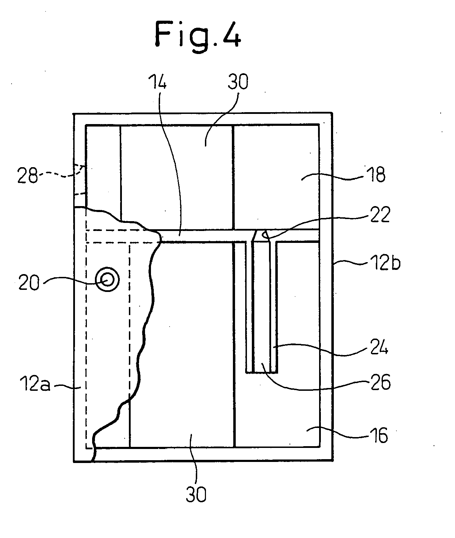

Further, dust filters 30 can be disposed, as shown in Fig. 4, in the expansion chamber 16 and the front chamber 18. For example, the dust filter 30 is arranged in the expansion chamber 16 or the front chamber 18 before welding the box portion 12a and the lid portion 12b of the housing 12 to each other. Alternatively, filter insertion openings (not shown) may be formed in the wall of the expansion chamber 16 and the front chamber 18, so that the dust filters 30 can be inserted therein or exchanged even after welding. By installing the dust filters 30 in the expansion chamber 16 and the front chamber 18 in this way, the silencer 12 can achieve the dust prevention effect and the silencing effect at the same time. As compared with a case in which a dust filter 30 is disposed separately from the silencer 10, the silencer 10 shown in Fig. 4 contributes to the saving of the space in a point that installation space for the dust filter 30 can be saved and at the same time prevents the increase of the pressure loss. In order to realize the dust prevention function, the dust filter 30 is required to be disposed at least in the expansion chamber 16 but not necessarily in the front chamber 18. However, in order realize a more excellent dust prevention function, it is preferable that the dust filters 30 are disposed in both the expansion chamber 16 and the front chamber 18.

When the dust filter 30 is arranged in the expansion chamber 16, it is only necessary to be arranged so as to obstruct a space between the suction port 20 and the rib 24, as shown in Fig. 4. Similarly, when the dust filter 30 is arranged in the front chamber 18, it is only necessary to be arranged so as to obstruct a space between the exhaust port 22 and the external communication port 28. A dust filter having an inner portion formed of glass fiber and an outer peripheral portion formed of foamed urethane is preferably used as the dust filter 30. However, other types of dust filters may also be used.

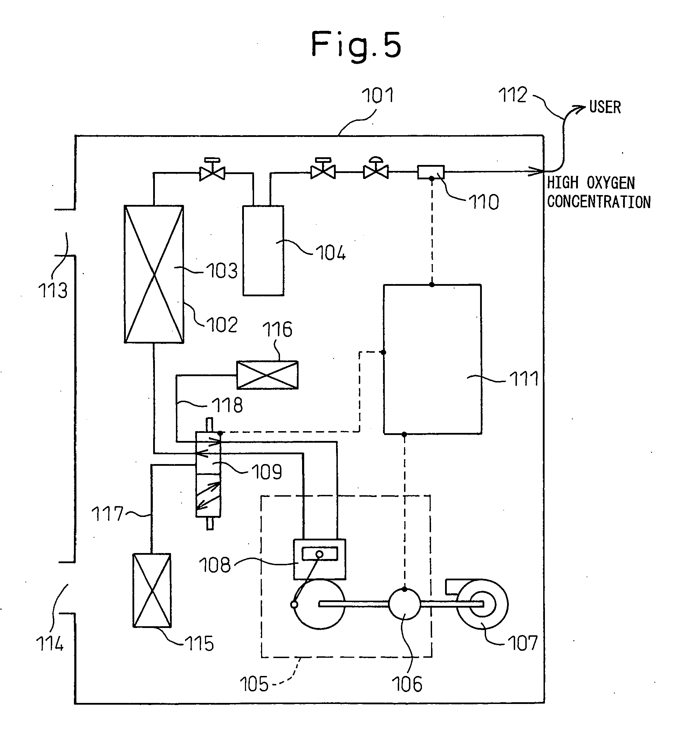

Next, with reference to Fig. 5, a general configuration of a variable-pressure-type oxygen concentrator equipped with the silencer 10 according to the present invention will be described. The variable-pressure-type oxygen concentrator 101 includes an adsorption cylinder 102 filled with an adsorbent 103 for selectively adsorbing nitrogen rather than oxygen, a product tank 104 for storing oxygen not adsorbed by the adsorption cylinder 102, a compressor 105, a flow passage switch valve 109 for switching a direction of air flow between the compressor 105 and the adsorption cylinder 102, a controller 111 for controlling the operations of the compressor 105 and the flow passage switch valve 109, a nose cannula 112 for supplying concentrated oxygen stored in the product tank 104 to a user, an exhaust-side silencer 115 connected to the purge side of the flow passage switch valve 109 and a suction-side silencer 116 connected to the intake side of the flow passage switch valve 109.

In the variable-pressure-type oxygen concentrator 101, an air introduced through the supply pipe 118 connected to the suction-side silencer 116 is pressurized by the compressor 105 and supplied to the adsorption cylinder 102. In the adsorption cylinder 102 in pressurized state, the nitrogen in the air is selectively adsorbed by the adsorbent 103 filled in the adsorption cylinder 102, and the oxygen not adsorbed is taken out of the adsorption cylinder 102 and stored in the product tank 104 as concentrated oxygen. Then, in accordance with an oxygen supply amount set by a flow rate setter 110, the concentrated oxygen is supplied to the user from the product tank 104 through the nose cannula 112.

The amount of nitrogen that can be adsorbed in one process is determined by the amount and type of the adsorbent 103. Therefore, before the amount of nitrogen adsorbed by the adsorbent 103 reaches a saturated amount, the flow passage is switched by the flow passage switch valve 109, and the interior of the adsorption cylinder 102 is decompressed by the compressor 105 thereby to regenerate the adsorbent 103 by desorbing the nitrogen. At the same time, the gas sucked in from the compressor 105 is discharged into the atmosphere through the flow passage switch valve 109, the exhaust pipe 117 connected to the purge side thereof and the exhaust-side silencer 115 connected to the exhaust pipe 117. The flow passage switch valve 109 is controlled by the controller 111 so as to be switched at predetermined time intervals. In order to increase the amount of adsorption and desorption in one process, the interior of the adsorption cylinder 102 may be vacuumized using the vacuum pump during the desorption process.

The compressor 105 includes a compressor drive motor 106 for driving the compressor 105 and a compressor compression mechanism unit 108. The compressor drive motor 106 rotationally drives the compressor 105 by rotating at a rotational speed set by the controller 111. The compressor compression mechanism unit 108 compresses the air using the turning force obtained by the compressor drive motor 106, and various types of compressor compression mechanisms exist according to the compression ways. The compressor compression mechanism unit 108 normally used for the oxygen concentrator 101 includes a reciprocating piston type and a rotary scroll type. However, as long as the air in the atmosphere can be compressed, any other type of compressor compression mechanism unit 108 can be used. Such a compressor 105 includes at least one supply air inlet port and at least one compression outlet port, which are connected to the adsorption cylinder or an appropriate place in the device, respectively. Further, the compressor 105 is cooled by a cooling fan 107.

The suction-side silencer 116 provided on a suction line of the compressor 105 is adapted to introduce the air from a suction duct connected to the suction port 20 and is connected to the compressor 105 through the supply pipe 118 extending from the front chamber 18. As the suction-side silencer 116, the silencer 10 having the dust filter 30 in the expansion chamber 16 is used. On the other hand, the exhaust-side silencer 115 arranged on a vacuum exhaust line of the compressor is connected to the exhaust side of the compressor 105 through the exhaust pipe 117 connected to the suction port 20 of the silencer 115. Since the dust prevention function is not indispensable for the exhaust-side silencer 115, the silencer 10 having no dust filter in the expansion chamber 16 is used as the exhaust-side silencer 115. Further, the front chamber 18 has two external communication ports 28 for discharging the exhaust gas containing nitrogen as a main component into the compressor box through the exhaust duct connected to the external communication passage 28.

Please note that the parts designated by reference numerals 113, 114 are supply/exhaust ports.

## Claims

1. An expansion-type silencer comprising a substantially rectangular parallelepiped-shaped expansion chamber surrounded by six partition walls and having a suction port and an exhaust port which are formed in the partition walls,
wherein said silencer further comprises a rib extending from one of the partition walls of the expansion chamber formed with the exhaust port toward the interior of the expansion chamber while being in contact with both of a pair of parallel partition walls adjacent to the one of the partition walls, said rib formed with an air passage for providing communication between the exhaust port and the expansion chamber.

2. The silencer according to claim 1, wherein the expansion chamber is formed by a box portion having four side walls, a bottom wall and the rib integrated with each other, and a lid portion closely contacting the four side walls and the rib, and wherein the air passage is formed by a groove formed on the rib and the lid portion closely contacting the rib.

3. The silencer according to claim 1, wherein one or both of the suction port and the exhaust port are formed by an orifice having a sectional area progressively decreasing along a direction of flow.

4. The silencer according to claim 1, wherein a front chamber having a communication port communicating with the outside is provided outside the exhaust port of the expansion chamber.

5. The silencer according to claim 1, further comprising a filter in the expansion chamber.

6. The silencer according to claim 4, further comprising a filter in the front chamber.

7. An expansion-type silencer comprising a substantially rectangular parallelepiped-shaped internal chamber surrounded by six partition walls, said internal chamber divided into a first chamber and a second chamber by a separation wall, said first chamber formed at an outer wall thereof with a suction port, said separation wall formed with an exhaust port,
wherein said silencer further comprising a rib extending from the separation wall formed with the exhaust port toward the interior of the first chamber while being in contact with both of a pair of parallel partition walls adjacent to the separation wall, said rib formed with an air passage connecting to the exhaust port for providing communication between the first and second chambers.

8. An oxygen concentrator comprising a supply pipe, an exhaust pipe and an expansion-type silencer connected to one or both of the supply pipe and the exhaust pipe, said oxygen concentrator separating oxygen from raw air supplied from the supply pipe thereby to generate an oxygen-concentrated gas and discharging exhaust gas after separation of oxygen from the exhaust pipe,
wherein said expansion-type silencer comprises a substantially rectangular parallelepiped-shaped expansion chamber surrounded by six partition walls and having a suction port and an exhaust port which are formed in the partition walls, and a rib extending from one of the partition walls of the expansion chamber formed with the exhaust port toward the interior of the expansion chamber while being in contact with both of a pair of parallel partition walls adjacent to the one of the partition walls, said rib formed with an air passage for providing communication between the exhaust port and the expansion chamber.

9. The oxygen concentrator according to claim 8, wherein the expansion chamber is formed by a box portion having four side walls, a bottom wall and the rib integrated with each other, and a lid portion closely contacting the four side walls and the rib, and wherein the air passage is formed by a groove formed on the rib and the lid portion closely contacting the rib.

10. The oxygen concentrator according to claim 8, wherein one or both of the suction port and the exhaust port are formed by an orifice having a sectional area progressively decreasing along a direction of flow.

11. The oxygen concentrator according to claim 8, wherein a front chamber having a communication port communicating with the outside is provided outside the exhaust port of the expansion chamber.

12. The oxygen concentrator according to claim 8, wherein the silencer further comprises a filter in the expansion chamber.

13. The oxygen concentrator according to claim 11, wherein the silencer further comprises a filter in the front chamber.
